# EUROPEAN PATENT APPLICATION

(11) **EP 4 260 843 A1**
(43) Date of publication of application: **18.10.2023**
(21) Application number: 22382351.9
(22) Date of filing: 13.04.2022
(51) Int. Cl.: A61K 8/37, A61K 8/60, A61Q 5/02, A61Q 19/10, C11D 1/06, C11D 3/20

(54) **COMPOSITION COMPRISING AN ALKYL AND/OR ALKENYLGLYCOSIDE DERIVATIVE AND AN ESTER DERIVED FROM A HYDROXYCARBOXYLIC ACID AND A FATTY ALCOHOL**

(71) Applicant: Kao Corporation S.A.U, 08210 Barberà del Vallès Barcelona (ES)
(72) Inventor: MARIMON MARGARIT, Núria, E-08210 Barberà del Vallès (Barcelona) (ES); NOGUÉS LÓPEZ, Blanca, E-08210 Barberà del Vallès (Barcelona) (ES)
(74) Representative: ABG Intellectual Property Law, S.L.

(57) **Abstract**

The present invention relates to a composition comprising an alkyl and/or alkenylglycoside derivative and an ester derived from a hydroxycarboxylic acid and a fatty alcohol, to a method for the preparation of said composition, cosmetic compositions which comprise said composition and to the use of said cosmetic composition in hair and/or skin cleansing applications. In particular, the glycoside derivative is a sugar moiety, i.e. a saccharide, derivatized by bonding one of its hydroxyl groups to an alkyl or alkenyl group forming an ether bond and one or more of the remaining hydroxyl groups to a moiety comprising an acid group or a salt thereof such as carboxylic acid or sulfonic acid or their salts. Specific examples are D-glucopyranose, oligomeric, C10-16-alkyl glycosides, carboxymethyl esters, sodium salts and sodium lauryl glucose carboxylate. Examples of the ester are lauryl lactate and myristyl lactate.

## Description

### FIELD OF THE INVENTION

The present invention relates to a composition comprising an alkyl and/or alkenylglycoside derivative and a fatty acid ester derived from a hydroxycarboxylic acid and a fatty alcohol, to a method for the preparation of said composition, the cosmetic compositions which comprises said composition and to the use of said cosmetic composition in hair and/or skin cleansing applications.

### STATE OF THE ART

Skin and hair cleanser compositions are required to have not only good detergency and cleansing ability, but they also need to be mild and be well tolerated by the skin and/or hair and do not cause excessive defatting or dryness to the skin and hair, with reduced tackiness, homogeneous, to be of easy applicability and to exhibit good foaming properties, such as foam stability, foam quantity, foam quality and foam smoothness. In addition, skin and hair cleanser compositions are demanded by consumers to exhibit good viscosity as well as good rheological properties of stability, feel and flow. Also, there is a concern in the customer products market towards the sustainability of cosmetic products. Thus, there is also a requirement to formulate products with ingredients that are considered "green" or "natural" since they are derived from renewable and/or sustainable sources.

The use of anionic surfactants in skin and hair cleanser formulations is well known by the persons skilled in the art.

EP1385612 describes the use of a surfactant composition containing a surfactant mixture having an alkyl and/or alkenyl oligoglycoside and an alkyl and/or alkenyl oligoglycoside ether carboxylic acid. This patent application describes aqueous compositions wherein the foam requirements and tackiness requirements are fulfilled.

EP3049051 describes cosmetic composition comprising a combination of surfactants of carboxylate, acylisethionate and alkyl(poly)glycoside type. This patent application describes compositions for the cosmetic treatment of keratin materials, and in particular for washing the hair with good foaming power.

From the state of the art set forth above, it can be seen that there is still a need for further compositions comprising an alkyl and/or alkenylglycoside derivatives for skin and/or hair cleansing.

### SUMMARY OF THE INVENTION

The present inventions aims at addressing the above-described needs, and provides a composition comprising an alkyl and/or alkenylglycoside derivative and a fatty acid ester derived from hydroxycarboxylic acid and a fatty alcohol, a process for the preparation of said composition; and its use in cosmetic applications for skin and/or hair cleansing. The compositions are able to comply with the requirements imposed on them and provide good performance. In particular, the compositions of the invention exhibit good viscosity, good rheological properties of stability, feel and flow, good foaming properties, such as foam stability, foam quantity, foam quality and foam smoothness. The compositions of the invention do also have good detergency and cleansing ability, they are mild and be well tolerated by the skin and/or hair and do not cause excessive defatting or dryness to the skin and hair, they also have reduced tackiness, they are homogeneous and easy applicable.

Thus, the first aspect of the present invention is related to a composition comprising:
a) at least an alkyl and/or alkenylglycoside derivative, and
b) at least a fatty acid ester derived from a hydroxycarboxylic acid and a fatty alcohol.

Processes for the preparation of the composition according to the first object are also an object of the invention.

The cosmetic composition comprising the composition of the invention is also an aspect of the invention.

Another aspect of the present invention is the use of a cosmetic composition of the invention for the care of skin and/or hair, in particular for cleansing skin and/or hair.

It is also an aspect of the present invention a process of preparation of the cleansing composition. The use of the cosmetic composition according to the invention for the cleansing treatment of skin and/or hair and a method of cleansing or conditioning skin and/or hair with the cosmetic composition are also aspects of the present invention.

### DETAILED DESCRIPTION OF THE INVENTION

All percentages are weight percentages, unless otherwise indicated.

As cosmetic composition it is understood a composition suitable as an ingredient to be used in cosmetic and personal care applications, in particular for skin and hair care.

The main object of the present invention is a composition comprising:
a) at least an alkyl and/or alkenylglycoside derivative, and
b) at least a fatty acid ester derived from a hydroxycarboxylic acid and a fatty alcohol.

### Alkyl and/ or alkenylglycoside derivative (a):

The composition of the present invention comprises a component (a), said component being an alkyl and/or alkenylglycoside derivative.

The terms "alkylglycoside derivative" and "alkenylglycoside derivative" refer to a sugar moiety (i.e. a saccharide, which may be a mono or oligosaccharide) which is derivatized by bonding of one of its hydroxyl groups to an alkyl or alkenyl group forming an ether bond and wherein one or more of the remaining hydroxyl groups of the sugar moiety is/are each bonded to a moiety which at least comprises an acid group or a salt thereof, such as carboxylic acid or sulfonic acid or their salts.

Examples of alkylglycoside derivatives and alkenylglycoside derivatives are described in EP1385612 and EP3049051, which are incorporated herein by reference.

In one embodiment, the composition comprises an alkylglycoside derivative. In another embodiment the composition comprises and alkenylglycoside derivative. In a further embodiment the composition comprises and alkylglycoside derivative and an alkenylglycoside derivative.

In an embodiment of the present invention, the alkyl and/or alkenylglycoside derivative is represented by formula (I):

R¹-[G]ₚ-[A]ₙ Formula (I)

wherein
R¹ represents an alkyl or alkenyl group having from 4 to 24 carbon atoms, preferably from 10 to 18 carbon atoms, more preferably from 10 to 16 carbon atoms, even more preferably from 12 to 16 carbon atoms;
G is a rest derived from a sugar unit containing 5 or 6 carbon atoms,
p represents a number within the range of 1 to 10,
n is a number from 1 to 4, and
A is selected from the group consisting of -(CH₂)ₘ-COOX, -C(=O)-CH₂-C(OH)(COOX)-CH₂-COOX, -C(=O)-CH(OH)-CH(OH)-COOX, -CH₂-CH(OH)-CH₂-SO₃X, -CH₂-CH(OH)-CH₂-C(=O)-CH₂-C(OH)(COOX)-CH₂-COOX, - P(O)(OX)₂, -CH₂-CH₂-SO₃X, and mixtures thereof, wherein m is a number from 1 to 5, and X is selected from the group consisting of hydrogen, an alkali metal, ammonium and alkaline earth metal.

The G group is a rest derived from a sugar unit containing 5 or 6 carbon atoms, preferably a rest derived from glucose. The bonding of the R¹- moiety is bonded to the sugar rest G by formation of an ether bond between R¹ and one hydroxyl group of the sugar unit G, the bonding of two G groups (-G-G-) when p>1 represents the formation of a glycosidic linkage, i.e. an ether bond between one hydroxyl group from one of the sugar units G and one hydroxyl group from the other sugar unit G (like in any disaccharide, which results in the loss of a hydrogen atom from one sugar unit G and a hydroxyl group from the other sugar unit G), and the bonding of the sugar unit G to the A moiety represents the formation of an ether bond between one hydroxyl group of the sugar unit G (implying the loss of the hydrogen atom of this hydroxyl group) and a carbon atom of the A moiety. Accordingly, -[G]ₚ- represents a radical derived from p sugar units G containing each 5 or 6 carbon atoms as defined herein but without n+1 of its -OH groups. A non-limiting example of a compound of formula (I) is shown below for a sugar unit G being glucose and wherein p is 1 and n is 1.

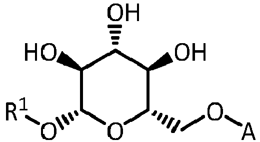

The alkyl or alkenylglycoside can be derived from aldoses or ketoses containing 5 or 6 carbon atoms, i.e. G is a rest derived from aldoses or ketoses containing 5 or 6 carbon atoms.

Examples of aldoses containing 5 or 6 carbon atoms are ribose, arabinose, xylose, lyxose and glucose. Examples of ketoses containing 5 or 6 carbon atoms are ribulose, xylulose, fructose, psocose, sorbose and tagatose.

Preferably, the alkyl or alkenylglycoside is derived from glucose, i.e. G is a rest derived from glucose.

The index p in the formula indicates the degree of oligomerization (i.e. the number of glycoside units, i.e. mono- and oligoglycosides), and is a number from 1 to 10. Whereas p in a given compound must always be an integer and, preferably, it may assume a value of 1 to 6, the p value for a certain alkyl and/or alkenylglycoside is an analytically determined quantity obtained by gas chromatography, which is generally not an integer but has decimals. In particular, alkyl and/or alkenylglycosides having an average degree of oligomerization p of 1.1 to 3.0.

In an embodiment of the present invention, R¹ is a linear or branched alkyl having from 4 to 24 carbon atoms or a linear alkenyl group having from 4 to 24 carbon atoms and from 1 to 3 double bonds; preferably the alkyl or alkenyl has from 10 to 18 carbon atoms, more preferably from 10 to 16 carbon atoms, even more preferably from 12 to 16 carbon atoms.

As used herein, the term "alkyl" refers to a straight or branched hydrocarbon chain having the indicated number of carbon atoms, preferably from 4 to 24 carbon atoms, more preferably from 10 to 18 carbon atoms, more preferably from 10 to 16 carbon atoms, even more preferably from 12 to 16 carbon atoms.

As used herein, the term "alkenyl" refers to a linear hydrocarbon chain having the indicated number of carbon atoms, preferably from 4 to 24 carbon atoms, more preferably from 10 to 18 carbon atoms, more preferably from 10 to 16 carbon atoms, even more preferably from 12 to 16 carbon atoms, and from 1 to 3 double bonds (i.e. 1, 2 or 3 double bonds).

In another embodiment of the present invention, R¹ is derived from primary alcohols having from 4 to 24 carbon atoms, preferably from 10 to 18 carbons atoms, more preferably from 10 to 16 carbon atoms, even more preferably from 12 to 16 carbon atoms, linear or branched, and optionally having from 1 to 3 double bonds. Examples of primary alcohols are capryl alcohol, pelargonic alcohol, capric alcohol, lauryl alcohol, myristyl alcohol, cetyl alcohol, palmitoleyl alcohol, stearyl alcohol, isostearyl alcohol, oleyl alcohol, linoleyl alcohol, petroselinyl alcohol, behenyl alcohol, erucyl alcohol and mixtures thereof. Thus, examples of R¹ groups are octanyl, nonanyl, decanyl, dodecanyl, tetradecanyl, hexadecanyl, hexadec-9-en-1-yl, octadecanyl, 16-methylheptadecan-1-yl, octadec-9-en-1-yl, octadeca-9,12-dien-1-yl, octadec-6-en-1-yl, docosanyl, docos-13-en-1-yl and mixtures thereof.

In a preferred embodiment of the present invention, R¹ is derived from lauryl alcohol, myristyl alcohol or mixtures thereof. Thus, in this preferred embodiment, R¹ is selected from dodecanyl, tetradecanyl and mixtures thereof.

In another embodiment of the present invention, the alcohols from which R¹ is derived are from vegetable origin.

In a more preferred embodiment, R¹ is derived from lauryl alcohol, i.e. R¹ is dodecanyl.

In another embodiment of the present invention, A is selected from the group consisting of -(CH₂)ₘ-COOX, -C(=O)-CH₂-C(OH)(COOX)-CH₂-COOX, -C(=O)-CH(OH)-CH(OH)-COOX, -CH₂-CH(OH)-CH₂-SO₃X, -CH₂-CH(OH)-CH₂-C(=O)-CH₂-C(OH)(COOX)-CH₂-COOX, and mixtures thereof, wherein m is a number from 1 to 5, preferably from 1 to 3, more preferably 1, and X is selected from the group consisting of hydrogen, an alkali metal, ammonium and alkaline earth metal, preferably sodium or hydrogen. In a preferred embodiment, A is - (CH₂)ₘ-COOX, wherein m is a number from 1 to 5, preferably from 1 to 3, more preferably 1, and X is selected from the group consisting of hydrogen, an alkali metal, ammonium and alkaline earth metal, preferably sodium or hydrogen. More preferably, A is -CH₂COOX, wherein X is alkali metal or hydrogen, preferably sodium or hydrogen, more preferably sodium.

In another embodiment of the present invention, the alkyl and/or alkenylglycoside derivative is an alkyl and/or alkenylglycoside carboxylate represented by formula (II)

R¹-[G]ₚ-[(CH₂)ₘ-COOX]ₙ Formula (II)

wherein R¹, G and p are as previously defined; G is a sugar unit containing 5 or 6 carbon atoms, p represents a number from 1 to 10, m is a number from 1 to 5, preferably from 1 to 3, more preferably 1, n is a number from 1 to 4, and X is selected from the group consisting of hydrogen, an alkali metal, ammonium and alkaline earth metal, preferably alkali metal and hydrogen, more preferably sodium.

As used herein, the term "alkali metal" refers to lithium, sodium, potassium, rubidium, caesium and francium, preferably sodium and/or potassium, more preferably sodium.

As used herein, the term "alkaline earth metal" refers to beryllium, magnesium, calcium and strontium, preferably magnesium and/or calcium.

The alkyl and/or alkenylglycoside derivative represented by formula (II) may be obtained by standard methods of preparative organic chemistry, for example, by reaction of alkyl and/or alkenylglycosides with halocarboxylic acids in alkaline medium.

The alkyl and/or alkenylglycoside carboxylate can be derived from aldoses or ketoses containing 5 or 6 carbon atoms, as previously defined, preferably glucose.

The index p in the formula indicates the degree of oligomerization (i.e. the number of glycoside units, i.e. of mono- and oligoglycosides), and is a number from 1 to 10. Whereas p in a given compound must always be an integer and, preferably, it may assume a value of 1 to 6, more preferably from 1 to 3, even more preferably 1 or 2, still more preferably 1, the p value for a certain alkyl and/or alkenylglycoside is an analytically determined calculated quantity which is generally a not an integer but has decimals. Alkyl and/or alkenylglycosides having an average degree of oligomerization p of 1.1 to 3.0.

m is a number from 1 to 5, preferably form 1 to 3, more preferably 1 or 2, even more preferably 1.

n is a number from 1 to 4.

X is selected from the group consisting of hydrogen, an alkali metal, ammonium and alkaline earth metal, preferably alkali metal can hydrogen, more preferably sodium and hydrogen, even more preferably sodium.

In an embodiment of the present invention, R¹ is a linear or branched alkyl having from 4 to 24 carbon atoms or a linear alkenyl group having from 4 to 24 carbon atoms and from 1 to 3 double bonds; preferably the alkyl or alkenyl has from 10 to 18 carbon atoms, more preferably from 10 to 16 carbon atoms, even more preferably from 12 to 16 carbon atoms.

The terms "alkyl" and "alkenyl" are as previously defined.

In another embodiment of the present invention, R¹ is derived from primary alcohols having from 4 to 24 carbon atoms, preferably from 10 to 18 carbons atoms, more preferably from 10 to 16 carbon atoms, even more preferably 12 to 16 carbon atoms. Examples of primary alcohols are capryl alcohol, pelargonic alcohol, capric alcohol, lauryl alcohol, myristyl alcohol, cetyl alcohol, palmitoleyl alcohol, stearyl alcohol, isostearyl alcohol, oleyl alcohol, linoleyl alcohol, petroselinyl alcohol, behenyl alcohol, erucyl alcohol and mixtures thereof. In a preferred embodiment of the present invention, R¹ is derived from lauryl alcohol, myristyl alcohol or mixtures thereof. In a more preferred embodiment, R¹ is derived from lauryl alcohol.

In a preferred embodiment of the present invention, the alkyl and/or alkenylglycoside derivative is represented by formula (II), wherein R¹ is a linear alkyl chain having from 10 to 16 carbon atoms, G represents a glucose, p is a number from 1 to 3, m is a number from 1 to 3, n is 1 to 4, and X represents an alkali metal. In a more preferred embodiment of the present invention, the alkyl and/or alkenylglycosidecarboxylate is a D-Glucopyranose, oligomeric, C10-16-alkyl glycosides, carboxymethyl ethers, sodium salts. In another preferred embodiment of the present invention, the alkyl and/or alkenylglycosidecarboxylate corresponds to component with INCI Name "Sodium Lauryl Glucose Carboxylate".

In an embodiment of the present invention, the amount of alkyl and/or alkenylglycoside derivative, i.e. component (a) in the composition of the invention is from 3 to 45% wt., preferably from 5 to 40% wt., more preferably from 6 to 35% wt., with respect to the sum of components (a), (b), (c) and (d).

### Fatty acid ester derived from hydroxycarboxylic acid and fatty alcohol (b):

The composition of the present invention comprises a component (b), said component being a fatty acid ester derived from a hydroxycarboxylic acid and a fatty alcohol. Thus, this component is an obtainable by esterification of a hydroxycarboxylic acid with a fatty alcohol.

As used herein, the term "hydroxycarboxylic acid" refers to a carboxylic acid having one or more hydroxyl substituents, preferably an alphahydroxycarboxylic acid.

As used herein, the term "fatty alcohol" refers to long chain alcohols, in particular long chain primary alcohols.

In an embodiment of the present invention, the hydroxycarboxylic acid is a C2-C8 alkyl or alkenyl group with an hydroxyl group attached to the carbon chain of the alkyl or alkenyl group, bonded to a COOH moiety. In a preferred embodiment, the hydroxycarboxylic acid is an alphahydroxycarboxylic acid, wherein the hydroxyl group is attached to the carbon atom next to the carboxyl group. Suitable hydroxycarboxylic acids include glycolic acid, lactic and citric acid.

In another embodiment of the present invention, the fatty alcohol is a C6 to C24 alcohol or mixture of C6 to C24 alcohol, preferably a C10 to C20 alcohol or mixture of C10 to C20 alcohol, more preferably a C10 to C16 alcohol or mixture of C10 to C16 alcohol, even more preferably a C12 to C16 alcohol or mixture of C12 to C16 alcohol. In particular, the fatty alcohol is a primary alcohol. Suitable fatty alcohols include capryl alcohol, pelargonic alcohol, capric alcohol, lauryl alcohol, myristyl alcohol, cetyl alcohol, palmitoleyl alcohol, stearyl alcohol, isostearyl alcohol, oleyl alcohol, linoleyl alcohol, petroselinyl alcohol, behenyl alcohol, erucyl alcohol and mixtures thereof, preferably lauryl alcohol.

In a preferred embodiment of the present invention, the fatty acid ester derived from a hydroxycarboxylic acid and a fatty alcohol is represented by formula (III):

R³⁻COO-R⁴ Formula (III)

R³ represents a hydroxyl-substituted alkyl or alkenyl group having 1 to 8 carbon atoms, optionally substituted by one or more -COOH groups, preferably wherein the hydroxyl group is attached to the carbon atom which is bonded to the -COO- moiety in formula (III). In a particular embodiment, R³ is a hydroxyl-substituted alkyl group having 1 to 4 carbon atoms, optionally substituted by one or more -COOH groups, preferably wherein the hydroxyl group is attached to the carbon atom which is bonded to the -COO- moiety in formula (III). Preferably R³ is selected from the group consisting of CH₂(OH)- (i.e. derived from glycolic acid), CH₃-CH₂(OH)- (i.e. derived from lactic acid) and COOH-CH₂-CH(CH₂-COOH)(OH)- (i.e. derived from citric acid), more preferably CH₃-CH₂(OH)- (i.e. derived from lactic acid).

R⁴ represents an alkyl or alkenyl group having from 6 to 24 carbon atoms, preferably from 10 to 20 carbon atoms, more preferably from 10 to 16, even more preferably 12 to 16 carbon atoms. Preferably, the fatty alcohol moiety is derived from a fatty alcohol selected from the group consisting of capryl alcohol, pelargonic alcohol, capric alcohol, lauryl alcohol, myristyl alcohol, cetyl alcohol, palmitoleyl alcohol, stearyl alcohol, isostearyl alcohol, oleyl alcohol, linoleyl alcohol, petroselinyl alcohol, behenyl alcohol, erucyl alcohol and mixtures thereof, preferably lauryl alcohol. Thus, preferably R⁴ is selected from the group consisting of octyl, nonyl, decyl, dodecyl, tetradecyl, hexadecyl, hexadec-9-en-1-yl, octadecanyl, 16-methylheptadecan-1-yl, octadec-9-en-1-yl, octadeca-9,12-dien-1-yl, octadec-6-en-1-yl, docosanyl, docos-13-en-1-yl, and mixtures thereof, preferably dodecyl.

In a preferred embodiment of the present invention, the fatty acid ester derived from hydroxycarboxylic acid and fatty alcohol is represented by formula (III), wherein the hydroxycarboxylic acid is lactic acid (i.e. R³ is CH₃-CH₂(OH)-), and the fatty alcohol moiety is selected from lauryl alcohol, myristyl alcohol, cetyl alcohol, capric alcohol, capryl alcohol and mixtures thereof, i.e. R⁴ is selected from the group consisting of dodecyl, tetradecyl, hexadecyl, decyl, octyl and mixtures thereof. In a more preferred embodiment of the present invention, the fatty acid ester derived from the hydroxycarboxylic acid and the fatty alcohol is selected from lauryl lactate and myristyl lactate, more preferably is lauryl lactate.

The fatty acid esters derived from the hydroxycarboxylic acid and the fatty alcohol are well known in the state of the art. Possible ways of obtention are described in patents EP0944572 or EP1802763.

In another embodiment of the present invention, the amount of fatty acid ester derived from a hydroxycarboxylic acid and a fatty alcohol, i.e. component (b) in the composition of the invention is from 2 to 20% wt., preferably from 2 to 16% wt., more preferably from 4 to 12% wt., with respect to the sum of components (a), (b), (c) and (d).

In a preferred embodiment, in the composition of the invention the ratio by weight of components (a) : (b) is from 98:2 to 2:98, preferably from 96:4 to 20:80, more preferably from 95:5 to 40:60.

### Alkyl and/ or alkenylglycoside (c):

The composition of the present invention may further comprise a component (c), said component being an alkyl and/or alkenylglycoside represented by formula (IV):

R¹-[G]ₚ Formula (IV)

wherein R¹, G and p are as defined for the compounds of formula (I) .

As explained for the compounds of formula (I), the alkyl or alkenylglycoside can be derived from aldoses or ketoses containing 5 or 6 carbon atoms, preferably glucose. The bonding of the R¹-moiety to the sugar rest G represents the formation of an ether bond between R¹ and one hydroxyl group of the sugar unit G, the bonding of two G groups (-G-G-) when p>1 represents the formation of a glycosidic linkage, i.e. an ether bond between one hydroxyl group from one of the sugar units G and one hydroxyl group from the other sugar unit G (like in any disaccharide, which results in the loss of a hydrogen atom from one sugar unit G and a hydroxyl group from the other sugar unit G). Accordingly -G represents a radical of the sugar unit containing 5 or 6 carbon atoms as defined herein but without one of its -OH groups. A non-limiting example of a compound of formula (IV) is shown below for a glucose sugar unit and wherein p is 1.

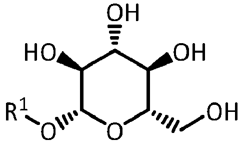

The index p is a number from 1 to 10. Whereas p in a given compound must always be an integer and, preferably, it may assume a value of 1 to 6, the value p for a certain alkyl and/or alkenylglycoside is an analytically determined calculated quantity which is generally not an integer but has decimals. In particular, alkyl and/or alkenylglycosides having an average degree of oligomerization p of 1.1 to 3.0.

In an embodiment of the present invention, R¹ is a linear or branched alkyl having from 4 to 24 carbon atoms or a linear alkenyl group having from 4 to 24 carbon atoms and from 1 to 3 double bonds; preferably the alkyl or alkenyl has from 10 to 18 carbon atoms, more preferably from 10 to 16 carbon atoms, even more preferably from 12 to 16 carbon atoms.

In another embodiment of the present invention, R¹ is derived from primary alcohols containing 4 to 24 carbon atoms, preferably containing 10 to 18 carbons atoms, more preferably 12 to 16 carbon atoms. Examples of primary alcohols are capryl alcohol, pelargonic alcohol, capric alcohol, lauryl alcohol, myristyl alcohol, cetyl alcohol, palmitoleyl alcohol, stearyl alcohol, isostearyl alcohol, oleyl alcohol, linoleyl alcohol, petroselinyl alcohol, behenyl alcohol, erucyl alcohol and mixtures thereof. Thus, examples of R¹ groups are octanyl, nonanyl, decanyl, dodecanyl, tetradecanyl, hexadecanyl, hexadec-9-en-1-yl, octadecanyl, 16-methylheptadecan-1-yl, octadec-9-en-1-yl, octadeca-9,12-dien-1-yl, octadec-6-en-1-yl, docosanyl, docos-13-en-1-yl and mixtures thereof.

In a preferred embodiment of the present invention, R1 is derived from lauryl alcohol, myristyl alcohol or mixtures thereof. Thus, in this preferred embodiment, R¹ is selected from dodecanyl, tetradecanyl and mixtures thereof.

In a more preferred embodiment, R¹ is derived from lauryl alcohol, i.e. R¹ is dodecanyl.

In another embodiment of the present invention, the alcohols from which R¹ is derived are from vegetable origin.

In an embodiment of the present invention, the alkyl and/or alkenylglycoside is represented by formula (IV), wherein R¹ is a linear alkyl chain having from 10 to 16 carbon atoms, G represents a glucose and p is a number from 1 to 3. In a preferred embodiment of the present invention, the alkyl and/or alkenylglycoside is a caprylyl/caprylglycoside, a myristylglycoside, a laurylglycoside a cocoglycoside and mixtures thereof. In a more preferred embodiment, the alkyl and/or alkenylglycoside is a laurylglycoside. In another preferred embodiment of the invention, the alkyl and/or alkenylglycoside is a D-Glucopyranose, oligomeric, C10-16-alkyl glycosides. In another preferred embodiment of the present invention, the alkyl and/or alkenylglycoside corresponds to component with INCI Name "Lauryl Glucoside".

In another embodiment of the present invention, the alkyl and/or alkenylglycoside is from natural origin (i.e. is derived from plants or vegetables).

The obtention of the alkyl and/or alkenylglycoside is well-known in the state of the art. Articles by Biermann et al. in Starch/Stärke 45, 281 (1993), B.Salka in Cosm. Toil. 108, 89 (1993) and J.Kahre et al. in SÖFW Journal No.8, 598 (1995) can be cited as representative of the extensive literature available on this component.

In an embodiment of the present invention, the alkyl and/or alkenylglycoside corresponds to the non-derivatived species from the obtention of the alkyl and/or alkenylglycoside derivative, (component (a)). In another embodiment, the alkyl and/or alkenylglycoside is different from the corresponing non-derivatived species from the obtention of the alkyl and/or alkenylglycoside derivative (component (a)).

In another embodiment of the present invention, the alkyl and/or alkenylglycoside corresponds to the non-carboxymethylated species from the obtention of the alkyl and/or alkenylglycoside derivative, (component (a)). In another embodiment, the alkyl and/or alkenylglycoside is different from the corresponding non-carboxymethylated species from the obtention of the alkyl and/or alkenylglycoside carboxylate (component (a)).

In another embodiment, the alkyl and/or alkenylglycoside is added to the composition as a blend or mixture.

In another embodiment of the invention, the amount of alkyl and/or alkenylgluycoside, i.e. component (c) in the composition is from 5 to 65 %wt., preferably from 10 to 60% wt., more preferably from 12 to 55 %wt., with respect to the sum of components (a), (b), (c) and (d).

In a preferred embodiment, in the composition of the invention the ratio by weight of components (a):(c) is from 95:5 to 5:95, preferably from 80:20 to 20:80, more preferably from 60:40 to 40:60.

In a particular embodiment, in the composition of the invention the ratio by weight of the sum of component (a) and component (c) to component (b) is from 98:2 to 31:69, preferably from 97:3 to 54:46, more preferably from 97:3 to 62:38.

### Amphoteric surfactant (d):

The composition of the present invention may further comprise an additional component (d), said component being an amphoteric surfactant.

Examples of amphoteric surfactant are alkyl amine oxides, alkyl betaines, alkyl sulphobetaines (sultaines), amidoalkyl betaines, alkyl glycinates, alkyl carboxyglycinates, alkyl amphoacetates, alkyl amphopropionates, alklylamphoglycinates, alkyl amidopropyl betaines, alkyl amidopropyl- and hydroxysultaines. Particularly preferred amphoteric surfactants are betaines, alkyl amine oxides, alkylamphoglycinates and alkyl amphoacetates.

In a preferred embodiment of the present invention, component (d) comprises one or more ampholytes. Specific examples of ampholytes are amine oxides. Suitable amine oxides according to the present invention are amine oxides with a hydrocarbon chain containing between 8 and 18 carbon atoms. Examples of commercially available amine oxides are those with the commercial reference OXIDET^{®} DM-20 (INCI name Lauramine Oxide) , OXIDET^{®} DMCLD (INCI name Cocamine Oxide)OXIDET^{®} DM-246 (INCI name Cocamine Oxide), OXIDET^{®} DM-4 (INCI name Myristamine Oxide), OXIDET^{®} L-75 (INCI name Cocamidopropylamine Oxide), all of them marketed by KAO Chemicals Europe.

In a most preferred embodiment of the present invention, component (d) comprises one or more betaines. Specific examples of betaines are alkyl betaines, alkyl sulphobetaines (sultaines), amidoalkyl betaines, alkyl glycinates, alkyl carboxyglycinates, alkyl amphoacetates, alkyl amphopropionates, alkylamphoglycinates, alkyl amidopropyl betaines and hydroxysultaines. Particularly preferred betaines are alkyl amidopropyl betaines, alkyl amidopropyl hydroxysultaines, alkyl hydroxysultaines and alkyl amphoacetates. In a preferred embodiment the betaines are alkyl amidopropyl betaines.

In the context of component (d), the term "alkyl" refers to a straight or branched hydrocarbon chain having no unsaturations and having form 8 to 18 carbon atoms.

Examples of alkyl amidopropyl betaines are cocamidopropyl betaine, behenamidopropyl betaine, capryl/capramidopropyl betaine, lauramidopropyl betaine, myristamidopropyl betaine, palmamidopropyl betaine, palmitamidopropyl betaine and mixtures thereof.

Examples of commercially available useful betaine surfactants according to the invention are BETADET^{®} HR, BETADET^{®} HR-50K, BETADET^{®} S-20, BETADET^{®} SHR and BETADET^{®} THC-2, all marketed by Kao Chemicals Europe.

In another embodiment of the invention, the amount of amphoteric surfactant, i.e. component (d) in the composition is from 15 to 75 %wt., preferably from 25 to 70% wt., more preferably from 30 to 65 %wt. with respect to the sum of components (a), (b), (c) and (d).

In a preferred embodiment, the ratio by weight of the sum of component (a) and component(c) to component (d) is from 90:10 to 10:90, preferably from 75:25 to 25:75, more preferably from 65:35 to 35:65.

In a particular embodiment, the ratio by weight of the sum of component (a) and component(c) to component (b) and to component (d), i.e. (a+c):(b):(d), is from 88:2:10 to 8:18:74, preferably from 74:2:25 to 21:18:62, more preferably from 64:2:34 to 29:18:53.

### Electrolyte (e)

The composition of the present invention may further comprise an additional component (e), said component being an electrolyte.

The electrolyte is preferably a mono-, di- or trivalent metal salt, and more particularly alkaline or alkaline-earth metal salts such as barium, calcium, sodium, potassium, magnesium salts and mixtures thereof. The anions constituting these salts may be chosen from carbonates, bicarbonates, sulfates, phosphates, sulfonates, glycerophosphates, borates, bromides, chlorides, nitrates, acetates, hydroxides.

Preferred electrolytes are magnesium chloride, potassium chloride, sodium chloride, calcium chloride, magnesium bromide, magnesium sulphate or magnesium chloride. Even more preferably, the electrolyte is sodium chloride.

In an embodiment of the invention, the electrolyte corresponds to the electrolyte species already present as a subproduct in component (a) and/or component (d). In another embodiment, the electrolyte is added to the composition as a blend or mixture.

In one embodiment of the invention, the amount of electrolyte, i.e. component (e) in the composition is from 10 to 30%wt., preferably from 10 to 25% wt., more preferably from 10 to 20 %wt. with respect to the total weight of the sum of components (a), (b), (c), (d) and (e).

### Composition of the invention:

The main object of the present invention has been described above and is a composition comprising:
a) at least an alkyl and/or alkenylglycoside derivative, and
b) at least a fatty acid ester derived from a hydroxycarboxylic acid and a fatty alcohol.

Other components of the composition of the invention are alkyl and/or alkenylglycoside (c), amphoteric surfactant (d), electrolyte (e) as previously defined.

In a particular embodiment, the composition comprises at least an alkyl and/or alkenylglycoside derivative (component (a)), and at least a fatty acid ester derived from a hydroxycarboxylic acid and a fatty alcohol (component (b)).

In another particular embodiment, the composition comprises at least an alkyl and/or alkenylglycoside derivative (component (a)), at least a fatty acid ester derived from a hydroxycarboxylic acid and a fatty alcohol (component (b)), and at least an alkyl and/or alkenylglycoside (component (c)).

In another particular embodiment, the composition comprises at least an alkyl and/or alkenylglycoside derivative (component (a)), at least a fatty acid ester derived from a hydroxycarboxylic acid and a fatty alcohol (component (b)), at least an alkyl and/or alkenylglycoside (component (c)), and at least an amphoteric surfactant (component (d)).

In one embodiment, in the composition of the invention:
- component (a) is a compound of formula (II), wherein R¹ is a linear alkyl chain having from 10 to 16 carbon atoms, G represents a glucose, p is a number from 1 to 3, m is a number from 1 to 3, n is 1 to 4, and X represents an alkali metal;
- component (b) is selected from lauryl lactate and myristyl lactate;
- component (c), when present, is selected from the group consisting of caprylyl/caprylglycoside, myristylglycoside, laurylglycoside, cocoglycoside and mixtures thereof; and
- component (d), when present, is an alkyl amidopropyl betaine.

In another embodiment, in the composition of the invention:
- component (a) is a compound of formula (II), wherein R¹ is a linear alkyl chain having from 10 to 16 carbon atoms, G represents a glucose, p is a number from 1 to 3, m is a number from 1 to 3, n is 1 to 4, and X represents an alkali metal;
- component (b) is selected from lauryl lactate and myristyl lactate;
- component (c), when present, is selected from the group consisting of caprylyl/caprylglycoside, myristylglycoside, laurylglycoside, cocoglycoside and mixtures thereof;
- component (d), when present, is an alkyl amidopropyl betaine;
- component (e), when present, is selected from the group consisting of magnesium chloride, potassium chloride, sodium chloride, calcium chloride, magnesium bromide, magnesium sulphate, magnesium chloride and mixtures thereof.

In another embodiment, in the composition of the invention:
- component (a) is sodium laurylglucose carboxylate;
- component (b) is lauryl lactate;
- component (c), when present, is laurylglycoside; and
- component (d), when present, is alkyl amidopropyl betaine.

In another embodiment, in the composition of the invention:
- component (a) is sodium laurylglucose carboxylate;
- component (b) is lauryl lactate;
- component (c), when present, is laurylglycoside;
- component (d), when present, is alkyl amidopropyl betaine; and
- component (e), when present, is sodium chloride.

In another embodiment, the composition comprises at least an alkyl and/or alkenylglycoside derivative (component (a)), at least a fatty acid ester derived from a hydroxycarboxylic acid and a fatty alcohol (component (b)), and at least an alkyl and/or alkenylglycoside (component (c)), wherein the active weight ratio between component (a) and component (c) is from 95:5 to 5:95, preferably from 80:20 to 20:80, more preferably from 60:40 to 40:60, wherein the active weight ratio among the sum of component (a) and component (c) and component (b), that is ((a) + (c)): (b) is from 98:2 to 31:69, preferably from 97:3 to 54:46, more preferably from 97:3 to 62:38.

In another embodiment, the composition comprises at least an alkyl and/or alkenylglycoside derivative (component (a)), at least a fatty acid ester derived from a hydroxycarboxylic acid and a fatty alcohol (component (b)), at least an alkyl and/or alkenylglycoside (component (c)),and at least an amphoteric surfactant (component (d)), wherein the active weight ratio among the sum of component (a) and component (c) and component (d), that is ((a)+(c)): (d) is from 90:10 to 10:90, preferably from 75:25 to 25:75, more preferably from 65:35 to 35:65; wherein the active weight ratio among the sum of component (a) and (c), component (b) and component (d), that is ((a)+(c)): (b): (d) is from 88:2:10 to 8:18:74, preferably from 74:2:25 to 21:18:62, more preferably from 64:2:34 to 29:18:53.

In another embodiment of the invention, the content of ethoxylated 1,4-dioxane in the composition is lower than 1 ppm.

In another embodiment of the invention, the content of diethanolamide in the composition is lower than 50 ppm.

The compositions may also comprise water.

### Process to obtain the composition:

The process for producing the composition of the invention comprises the steps of:
a. Mixing components (a) and (b) and, if present in the compositions, components (c), (d) and/or (e), with water at a temperature suitable for homogenization thereof, preferably at room temperature;
b. Stirring to obtain a homogeneous solution

Another process for producing the composition of the invention, comprises the steps of:
a. mixing components (a) and (b) and, if present in the compositions, components (c) and/or (d), without the presence of water to obtain a pre-mixture,
b. add the pre-mixture to water and stirring at a temperature suitable for homogeneization thereof.

Alternatively, the process for producing the composition of the invention comprises the steps of:
a. mixing component (a), component (b) and component (c) without the presence of water to obtain a pre-mixture, and
b. add the pre-mixture to water, optionally adding further components (d) and stirring at a temperature suitable for homogenization thereof.

### Cosmetic composition:

Another object of the present invention is a cosmetic composition comprising a component (a) comprising at least an alkyl and/or alkenylglycoside derivative, a component (b) comprising at least a fatty acid ester derived from a hydroxycarboxylic acid and a fatty alcohol, and additionally optionally further a component (c) comprising at least an alkyl and/or alkenylglycoside, optionally a component (d) comprising at least an amphoteric surfactant and optionally a component (e) comprising at least an electrolyte, and water.

In an embodiment of the present invention, the amount of alkyl and/or alkenylglycoside derivative, i.e. component (a) in the composition of the invention is from 0.1 to 20% wt. based on the total weight of the cosmetic composition, preferably from 0.3 to 15% wt., even more preferably from 0.3 to 13% wt.

In another embodiment of the present invention, the amount of fatty acid ester derived from a hydroxycarboxylic acid and a fatty alcohol , i.e. component (b) in the composition of the invention is from 0.1 to 8 % wt. %wt. based on the total weight of the cosmetic composition, preferably from 0.1 to 6% wt., more preferably from 0.2 to 5% wt.

In another embodiment of the invention, the amount of alkyl and/or alkenylgluycoside, i.e. component (c) in the composition is from 0.2 to 25 %wt. based on the total weight of the cosmetic composition, preferably from 0.4 to 20% wt., more preferably from 0.5 to 18 %wt.

In another embodiment of the invention, the amount of amphoteric surfactant, i.e. component (d) in the composition is from 0.7 to 30%wt. based on the total weight of the cosmetic composition, preferably from 0.8 to 25% wt., more preferably from 1 to 23 %wt.

In another embodiment of the invention, the amount of electrolyte, i.e. component (e) in the composition is from 0.5 to 15%wt. based on the total weight of the cosmetic composition, preferably from 0.5 to 13% wt., more preferably from 0.5 to 10 %wt.

In an embodiment of the invention, the cosmetic composition comprises:
- between 0.1 and 20%, preferably between 0.3% and 15%, more preferably between 0.3 and 13% wt. of component (a)
- between 0.1 and 8%, preferably between 0.1% and 6%, more preferably between 0.2 and 5 %wt. of component (b)
each of the indicated amounts being expressed as percentage by weight of the mentioned component with respect to the total weight of the composition.

In another embodiment of the invention, the cosmetic composition comprises:
- between 0.1 and 20%, preferably between 0.3% and 15%, more preferably between 0.3 and 13% wt. of component (a)
- between 0.1 and 8%, preferably between 0.1% and 6%, more preferably between 0.2 and 5 %wt. of component (b)
- between 0.2 and 25%, preferably between 0.4% and 20%, more preferably between 0.5 and 18% of component (c)
each of the indicated amounts being expressed as percentage by weight of the mentioned component with respect to the total weight of the composition.

In another embodiment of the invention, the cosmetic composition comprises:
- between 0.1 and 20%, preferably between 0.3% and 15%, more preferably between 0.3 and 13% wt. of component (a)
- between 0.1 and 8%, preferably between 0.1% and 6%, more preferably between 0.2 and 5 %wt. of component (b)
- between 0.2 and 25%, preferably between 0.4% and 20%, more preferably between 0.5 and 18% of component (c)
- between 0.7 and 30%, preferably between 0.8% and 25%, more preferably between 1 and 23% of component (d)
each of the indicated amounts being expressed as percentage by weight of the mentioned component with respect to the total weight of the composition.

In another embodiment of the invention, the cosmetic composition comprises:
- between 0.1 and 20%, preferably between 0.3% and 15%, more preferably between 0.3 and 13% wt. of component (a)
- between 0.1 and 8%, preferably between 0.1% and 6%, more preferably between 0.2 and 5 %wt. of component (b)
- between 0.2 and 25%, preferably between 0.4% and 20%, more preferably between 0.5 and 18% of component (c)
- between 0.7 and 30%, preferably between 0.8% and 25%, more preferably between 1 and 23% of component (d)
- between 0.5 and 15%, preferably between 0.5 and 13%, more preferably between 0.5 and 10% of component (e)
each of the indicated amounts being expressed as percentage by weight of the mentioned component with respect to the total weight of the composition.

In another embodiment of the present invention, the total amount of components (a), (b), (c) and (d), in the cosmetic composition is between 5 and 50% wt., preferably between 7 and 40% wt., more preferably between 10 and 35% wt. with respect to the total weight of the composition.

The pH of the cosmetic composition of the present invention is comprised between 3.5 and 7.5, preferably between 4.0 and 7.0, more preferably between 4.5 and 6.5

The cosmetic composition according to the present invention may also comprise oil components, silicone compounds, powders, nonionic surfactants, anionic surfactants, polymers, metal ion sequestering agents, UV protection factors, vitamins, antioxidants, antioxidant aids, perfume oils, germ inhibitors and the like as further auxiliaries and additives.

Examples of oils include liquid oils, solid oils, waxes, hydrocarbon oils and synthetic ester oils. Suitable oil components are, for example, Guerbet alcohols based on fatty alcohols containing 6 to 22 and preferably 8 to 10 carbon atoms, esters of linear C6-C22 fatty acids with linear C6-C22 fatty alcohols, esters of branched C6-C22 carboxylic acids with linear C6-C22 fatty alcohols such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isopropyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl cleats, erucyl behenate and erucyl erucate. Also suitable are esters of linear C6-C22 fatty acids with branched alcohols, more particularly 2-ethyl hexanol, esters of hydroxycarboxylic acids with linear or branched C6-C22 fatty alcohols, esters of linear and/or branched fatty acids with polyhydric alcohols for example propylene glycol, dimer diol or trimer triol) and/or Guerbet alcohols, triglycerides based on C6-C10 fatty acids, liquid mono-/di-/triglyceride mixtures based on C6-C18 fatty acids, esters of C6-C22 fatty alcohols and/or Guerbet alcohols with aromatic carboxylic acids, more particularly benzoic acid, esters of C6-C12 dicarboxylic acids with linear or branched alcohols containing 1 to 22 carbon atoms or polyols containing 2 to 10 carbon atoms and 2 to 6 hydroxyl groups, vegetable oils such as avocado oil, almond oil, hazelnut oil, babassu palm oil, borage oil, peanut oil, jojoba oil, canola oil, hemp oil, soybean oil, milk thistle oil, safflower oil, chufa oil, coconut oil, rapeseed oil, black cumin oil, wheat germ oil, sunflower oil, linseed oil, macadamia nut oil, corn oil, walnut oil, olive oil, branched primary alcohols, substituted cyclohexanes, linear and branched C6-C22 fatty alcohol carbonates, Guerbet carbonates, esters of benzoic acid with linear and/or branched C6-C22 alcohols, linear or branched, symmetrical or non-symmetrical dialkyl ethers containing 6 to 22 carbon atoms per alkyl group, ring opening products of epoxidized fatty acid esters with polyols, silicone oils and/or aliphatic or naphthenic hydrocarbons, for example dialkyl cyclohexanes.

Examples of waxes include natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes, microwaxes; chemically modified waxes (hard waxes) such as for example, montan ester waxes, sasol waxes, hydrogenated jojoba waxes and synthetic waxes such as, for example, polyalkylene waxes and polyethylene glycol waxes

Examples of hydrocarbon oils include liquid paraffin, squalane, pristane, paraffin, ceresin, squalene, petrolatum, and microcrystalline wax.

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Preferred silicone compounds are hydrophobic silicone oils, which are silicone oils which are soluble in paraffinic oil at 25°C. Hydrophobic silicone oils to be used according to the present invention include both volatile and non-volatile silicone oils.

Specific examples include a cyclic methyl siloxane having the formula {(CH₃)₂SiO}ₓ in which x is 3-6, or short chain linear methyl siloxanes having the formula ((CH₃)₂SiO{(CH₃)₂SiO}_{y}Si(CH₃)₃ in which y is 0-5.

Some suitable cyclic methyl siloxanes are hexamethylcyclotrisiloxanes (D3), a solid with a boiling point of 134°C and the formula {(Me₂)SiO}₃; octamethylcyclotetrasiloxane (D4) with a boiling point of 176°C, a viscosity of 2.3 mm²/s, and the formula {(Me₂)SiO}₄; decamethylcyclopentasiloxane (D5) (cyclomethicone) with a boiling point of 210°C, a viscosity of 3.87 mm²/s, and the formula {(Me₂)SiO}₅; and dodecamethylcyclohexasiloxane (DE) with a boiling point of 245°C, a viscosity of 6.62 mm²/s and the formula {(Me₂)SiO}₆.

Some suitable short linear methyl siloxane are hexamethyldisiloxane (MM) with a boiling point of 100°C, viscosity of 0-65 mm<2>/s, and formula Me₃SiOMe₃; octamethyltrisiloxane (MDM) with a boiling point of 152°C., viscosity of 1.04 mm²/s, and formula Me₃SiOMe₂SiOSiMe₃; decamethyltetrasiloxane (MD2M) with a boiling point of 194°C, viscosity of 1.53 mm²/s, and formula Me₃SiO(MeSiO)₂SiMe₃; dodecamethylpentasiloxane (MD3M) with a boiling point of 229°C, viscosity of 2.06 mm²/s, and formula Me₃SiO(Me₂SiO)₃SiMe₃; tetradecamethylhexasiloxane (MD4M) with a boiling point of 245°C, viscosity of 2.63 mm²/s, and formula Me₃SiO(Me₂SiO)₄SiMe₃; and hexadecamethylheptasiloxane (MD5M) with a boiling point of 270°C, viscosity of 3.24 mm²/s, and formula Me₃SiO(Me₂SiO)₅SiMe₃.

Furthermore, long chain linear siloxanes such as phenyltrimethicone, bis(phenylpropyl)dimethicone, dimethicone, and dimethiconol are also included.

Examples of powders include inorganic powders such as talc, kaolin, mica, sericite, muscovite, phlogopite, synthetic mica, lepidolite, biotite, vermiculite, bentonite, hectorite, laponite, magnesium carbonate, calcium carbonate, aluminum silicate, barium silicate, calcium silicate, magnesium silicate, strontium silicate, tungstate, magnesium, silica, zeolite, barium sulfate, calcined calcium sulfate (calcined gypsum), calcium phosphate, fluorine apatite, hydroxyapatite, ceramic powder, metallic soap (e.g., zinc myristate, calcium palimitate, and aluminum stearate), and boron nitride; organic powders such as polyamide resin powder (nylon powder), polyethylene powder, polymethylmethacrylate powder, polystyrene powder, styrene-acrylic acid copolymer resin powder, benzoguanamine resin powder, poly(tetrafluroethylene) powder, and cellulose powder; inorganic white pigments such as titanium dioxide and zinc oxide; inorganic red pigments such as iron oxide (red iron oxide) and iron titanate; inorganic brown pigments such as γ-iron oxide; inorganic yellow pigments such as yellow iron oxide and ocher; inorganic black pigments such as black iron oxide and lower order titanium oxide; inorganic purple pigments such as mango violet and cobalt violet; inorganic green pigments such as chrome oxide, chrome hydroxide, and cobalt titanate; inorganic blue pigments such as ultramarine and Prussian blue; pearl pigments such as titanium oxide coated mica, titanium oxide coated bismuth oxychloride, titanium oxide coated talc, colored titanium oxide coated mica, bismuth oxychloride, and fish scale flakes; metal powder pigments such as aluminum powder and copper powder; organic pigments such as zirconium, barium, or aluminum lake (e.g., organic pigments such as Red No.201, Red No.202, Red No.204, Red No.205, Red No.220, Red No.226, Red No.228, Red No.405, Orange No.203, Orange No.204, Yellow No.205, Yellow No.401, and Blue No.404,or Red No.3, Red No.104, Red No.106, Red No.227, Red No.230, Red No.401, Red No.505, Orange No.205, Yellow No.4, Yellow No.5, Yellow No.202, Yellow No.203, Green No.3, and Blue No.1); and natural colors such as chlorophyll and β-carotene.

Examples of lipophilic nonionic surfactants include sorbitan fatty acid esters (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate); glycerol or polyglycerol fatty acid esters (such as glycerol mono-cotton seed oil fatty acid ester, glycerol monoerucate, glycerol sesquioleate, glycerol monostearate, glycerol-a, α'-oleate pyroglutamate, and glycerol monostearate malate); propylene glycol fatty acid esters (such as propylene glycol monostearate); hardened castor oil derivatives; and glycerol alkyl ethers.

Examples of hydrophilic nonionic surfactants include POE-sorbitan fatty acid esters (such as POE-sorbitan monooleate, POE-sorbitan monostearate, and POE-sorbitan tetraoleate); POE sorbitol fatty acid esters (such as POE-sorbitol monolaurate, POE-sorbitol monooleate, POE-sorbitol pentaoleate, and POE-sorbitol monostearate); POE-glycerol fatty acid esters (such as POE-monooleates, POE-glycerol monostearate, POE-glycerol monoisostearate, and POE-glycerol triisostearate); POE-fatty acid esters (such as POE-distearate, POE-monodioleate, and ethylene glycol distearate); POE-alkyl ethers (such as POE-lauryl ether, POE-oleyl ether, POE-stearyl ether, POE-behenyl ether, POE-2-octyldodecyl ether, and POE-cholestanol ether); Pluronic type surfactants (such as Pluronic); POE/POP-alkyl ethers (such as POE/POP cetyl ether, POE/POP 2-decyltetradecyl ether, POE/POP monobutyl ether, POE/POP hydrogenated lanolin, and POE/POP glycerol ether); tetra POE/tetra POP-ethylenediamine condensates (such as Tetronic); POE-castor oil or hardened castor oil derivatives (such as POE-castor oil, POE-hardened castor oil, POE-hardened castor oil monoisostearate, POE- hardened castor oil triisostearate, POE-hardened castor oil monopyroglutamate monoisostearate diester, and POE-hardened castor oil maleate); POE-beeswax lanolin derivatives (such as POE-sorbitol beeswax); alkanolamides (such as coconut oil fatty acid diethanolamide, lauric acid monoethanolamide, and fatty acid isopropanolamide); POE-propylene glycol fatty acid esters; POE-alkylamines; POE-fatty acid amides; sucrose fatty acid esters; alkylethoxydimethylamine oxides; and trioleyl phosphate.

Examples of anionic surfactant, are, for example, alkyl sulphates (such as C6-C22 alkyl sulphates, metal salts of said C6-C22 alkyl sulphates as well as the ammonium salts or the salts of the organic amines with alkyl or hydroxyalkyl substituents), alkyl ether sulfate surfactant type (such as C6-C22 alkyl ether sulphates containing 0.5 to 5, preferably 0.8 to 3, moles of ethylene oxide per mol of the C6-C22 alkyl ether sulphate, metal salts of said alkylC6-C22 ether sulphates as well as the ammonium salts of organic amines with alkyl or hydroxyalkyl substituents, examples are sodium lauryl ether sulphate, potassium lauryl ether sulphate, ammonium lauryl ether sulphate and mono-, di- and triethylethanolamine lauryl ether sulphates containing from 0.8 to 3 moles of ethylene oxide per mole of alkyl ether sulphate, or mixtures thereof). Anionic surfactants of the sulfosuccinate types are also possible examples, including the alkylC6-C22 sulfosuccinates and alkylC6-C22 ether sulfosuccinates, preferably the mono- and di-alkylC6-C22 sulfosuccinates and mono- and di-alkylC6-C22 ether sulfosuccinates containing from 0.5 to 10, preferably from 1 to 5 mol of ethylene oxide per mol of alkyl (eg, mono- or di-alkyl) C6-C22 ether sulfosuccinate, or mixtures thereof, it being possible to use the metal salts of said mono- and di-alkylC6-C22 sulfosuccinates and mono - and di-alkyl C6-C22 ether sulfosuccinates as well as ammonium salts or salts of organic amines with alkyl or hydroxyalkyl substituents.

Suitable cationic polymers are, for example, cationic cellulose derivatives such as, for example, the quaternized hydroxyethyl cellulose obtainable from Amerchol under the name of Polymer JR 400, cationic starch, copolymers of diallyl ammonium salts and acrylamides, quaternized vinyl pyrrolidone/vinyl imidazole polymers such as, for example, Luviquat (BASF), condensation products of polyglycols and amines, quaternized collagen polypeptides such as, for example, Lauryidimonium Hydroxypropyl Hydrolyzed Collagen, quaternized wheat polypeptides, polyethyleneimine, cationic silicone polymers such as, for example, Amodimethicone, copolymers of adipic acid and dimethylamino-hydroxypropyl diethylenetriamine (Cartaretine, Sandoz), copolymers of acrylic acid with dimethyl diallyl ammonium chloride, polyquaternium type polymers, polyaminopolyamides and crosslinked water-soluble polymers thereof, cationic chitin derivatives such as, for example, quaternized chitosan, optionally in micro-crystalline distribution, condensation products of dihaloalkyls, for example dibromobutane, with bis-dialkylamines, for example bis-dimethylamino-1,3-propane, cationic guar gum such as, for example, Jaguar CBS, Jaguar C-17, Jaguar C-16 of Celanese, quaternized ammonium salt polymers such as, for example, Mirapol A-15, Mirapol AD-1, Mirapol AZ-1 of Mirapol.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl, acetate copolymers, vinyl pyrrolidone/dimethylaminoethyl methacrylate/vinyl caprolactam terpolymers and optionally derivatized cellulose ethers and silicones.

Examples of UV protection factors include organic substances (light filters) which are liquid or crystalline at room temperature and which are capable of absorbing ultraviolet radiation and of releasing the energy absorbed in the form of longer-wave radiation, for example heat. UV-B filters can be oil-soluble or water-soluble. The following are examples of oil-soluble substances: 3-benzylidene camphor or 3-benzylidene norcamphor and derivatives thereof, for example 3-(4-methylbenzylidene)-camphor; 4-aminobenzoic acid derivatives, preferably 4-(dimethylamino)-benzoic acid-2-ethylhexyl ester, 4-(dimethylamino)-benzoic acid-2-octyl ester and 4-(dimethylamino)-benzoic acid Amylester; esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene); esters of salicylic acid, preferably salicylic acid-2-ethylhexyl ester, salicylic acid-4-isopropylbenzyl ester, salicylic acid homomethyl ester; derivatives of benzophenone, preferably 2-hydroxy-4-methoxybenzo-phenone, 2-hydroxy-4-methoxy-4'-methylbenzophenone, 2,2'-dihydroxy-4-methoxybenzophenone; esters of benzalmalonic acid, preferably 4-methoxybenzalmalonic acid di-2-ethylhexyl ester; triazine derivatives such as, for example, 2,4,6-trianilino-(p-carbo-2'-ethyl-1'-hexyloxy)-1,3,5-triazine and Octyl Triazone; propane-1,3-diones such as, for example, 1-(4-tert.butylphenyl)-3-(4T-methoxyphenyl)-propane-1,3-dione; 2-phenylbenzimidazole-5-sulfonic acid and alkali metal, alkaline earth metal, ammonium, alkylammonium, alkanolammonium and glucammonium salts thereof; sulfonic acid derivatives of benzophenones, preferably 2-hydroxy-4-methoxybenzophenone-5-sulfonic acid and salts thereof; sulfonic acid derivatives of 3-benzylidene camphor such as, for example, 4-(2-oxo-3-bornylidenemethyl)-benzene sulfonic acid and 2-methyl-5-(2-oxo-3-bornylidene)-sulfonic acid and salts thereof.

Typical UV-A filters are, in particular, derivatives of benzoyl methane such as, for example 1-(4'-tert.butylphenyl)-3-(4'-methoxyphenyl)-propane-1,3-dione, 4-tert-butyl-4'-methoxydibenzoyl methane (Parsol 1789) or 1-phenyl-3-(4'-isopropylphenyl)-propane-1,3-dione.

The UV-A and UV-B filters may of course also be used in the form of mixtures. Besides the soluble substances mentioned, insoluble pigments, i.e. finely dispersed metal oxides or salts, may also be used for this purpose. Examples of suitable metal oxides are, in particular, zinc oxide and titanium dioxide and also oxides of iron, zirconium, silicon, manganese, aluminium and cerium and mixtures thereof. Silicates (talcum), barium sulfate and zinc stearate may be used as salts. The oxides and salts are used in the form of the pigments for skin-care and skin-protecting emulsions and decorative cosmetics. The particles should have an average diameter of less than 100 nm, preferably from 5 to 50 nm and more preferably from 15 to 30 nm. They may be spherical in shape although ellipsoidal particles or other non-spherical particles may also be used. The pigments may also be surface-treated, i.e. hydrophilicized or hydrophobicized. Typical examples are coated titanium dioxides such as, for example, Titandioxid T 805 (Degussa) or Eusolex T2000 (Merck). Suitable hydrophobic coating materials are, above all, silicones and especially trialkoxyoctyl silanes or simethicones. So-called micro- or nanopigments are preferably used in sun protection products. Micronized zinc oxide is preferably used.

Besides the two above-mentioned groups of primary protection factors, secondary protection factors of the antioxidant type may also be used. Secondary sun protection factors of the antioxidant type interrupt the photochemical reaction chain which is initiated when UV rays penetrate into the skin. Typical examples of suitable antioxidants are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example α-carotene, β-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, γ-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages (also (metal) chelators (for example (α-hydroxyfatty acids, palmitic acid, phytic acid, lactoferrine), α-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example γ-linolenic acid, linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, (α-glycosyl rutin, ferulic acid, furfurylidene glucitol, carnosine, butyl hydroxytoluene, butyl hydroxyanisole, nordihydroguaiac resin acid, nordihydroguaiaretic acid, trihydroxy-butyrophenone, uric acid and derivatives thereof, mannose and derivatives thereof, Superoxid-Dismutase, zinc and derivatives thereof (for example ZnO, ZnSO4), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Examples of metal ion sequestering agents include 1-hydroxyethane-1,1-diphosphonic acid, 1-hydroxyethane-1,1-diphosphonic acid 4Na salt, disodium edetate, trisodium edetate, tetrasorium edetate, sodium citrate, sodium polyphosphate, sodium metaphosphate, gluconic acid, phosphoric acid, citric acid, ascorbic acid, succinic acid, edetic acid, and trisodium hydroxyethyl ethylenediamine triacetate.

Examples of vitamins include vitamins A, B1, B2, B6, C, and E and the derivatives thereof; pantothenic acid and the derivatives thereof; and biotin.

Examples of antioxidants include tocopherols, dibutylhydroxytoluene, butylhydroxyanisole, and gallic acid esters. Examples of antioxidant aids include phosphoric acid, citric acid, ascorbic acid, maleic acid, malonic acid, succinic acid, lactic acid, fumaric acid, cephalin, hexametaphosphates, phytic acid, and ethylenediaminetetraacetic acid.

Suitable perfume oils are mixtures of natural and synthetic fragrances. Natural fragrances include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamon, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert-butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, filial and bourgeonal. Examples of suitable ketones are the ionones, α-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable fragrance. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, labolanum oil and lavendin oil. The following are preferably used either individually or in the farm of mixtures: bergamot oil, dihydromyrcenol, lilial, lyral, citronellol, phenylethyl alcohol, α-nexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, β-damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

Typical examples of germ inhibitors are preservatives which act specifically against gram-positive bacteria such as, for example, 2,4,4'-trichloro-2'-hydroxydiphenyl ether, chlorhexidine (1,6-di-(4-chlorophenyl-biguanido)-hexane) or TCC (3,4,4'-trichlorocarbanilide). Numerous perfumes and essential oils also have antimicrobial properties. Typical examples are the active substances eugenol, menthol and thymol in clove, mint and thyme oil.

The cosmetic compositions can be used in a large number of types of product for the skin and/or the hair such as mousses, gels, masks for the face or the hair, conditioners, formulations for improving hairstyling, or for facilitating the combing or disentangling of the hair, for providing volume or sheen, rinsing formulations, compositions for dying or colouring the hair, hand and body lotions and oils, products for improving the moisturization of the skin, cleansing milks, make-up-removing compositions, creams or lotions for protecting against the sun and ultraviolet radiation, care and/or treatment milks and creams, anti-acne preparations, local analgesics, mascaras, products intended to be applied to the lips or other mucous membranes, sticks, deodorant and antiperspirant products, shaving lotions, bath oils, talcs and other compositions of the same type.

In an embodiment of the invention, the compositions of the invention can be used as cosmetic compositions. In another embodiment, the compositions and cosmetic compositions of the invention are used for hair care and/or for skin care. In another embodiment, the compositions of the invention can be used in compositions for the cleansing of skin and/or hair, hair dyeing compositions, and compositions for conditioning and moisturizing of skin and/or hair.

Thus, a further aspect refers to the use of the cosmetic compositions of the invention for the care of skin and/or hair selected from the group consisting of cleansing of skin and/or hair, conditioning and moisturizing of skin and/or hair, and the dyeing of hair, preferably cleansing of skin and/or hair.

In a preferred embodiment, the cosmetic compositions of the invention are used for the cleansing of skin and/or hair.

A method of cleansing skin and/or hair is also part of the invention, wherein the method comprises the steps of wetting or dampening the skin and/or hair, applying the cosmetic composition according to the invention on the skin and/or hair, and rinsing the skin and/or hair with water.

A method of conditioning human hair and/or skin, wherein the method comprises the steps of applying the cosmetic composition according to the invention to wet hair and/or wet skin, and either rinse it off from the hair and/or skin with water, or alternatively be left on hair and/or skin, is also a part of the invention.

In particular, in the methods defined above a sufficient amount of the cosmetic composition of the invention is applied. The term "sufficient amount" refers to the amount of cosmetic composition necessary to achieve the cleansing or conditions of skin and/or hair and can be easily determined by the skilled person. An example of a sufficient amount is from 0.2 g to 20 g, more preferably from 0.5 g to 15 g.

The following examples are given in order to provide a person skilled in the art with a sufficiently clear and complete explanation of the present invention, but should not be considered as limiting of the essential aspects of its subject, as set out in the preceding portions of this description.

### EXAMPLES:

The first part of the Examples section corresponds to the preparation of the cosmetic compositions according to the invention. The second part of the Examples section refers to the performance of the cosmetic composition of the present invention.

### Example 1

### Preparation of Composition A (comparative)

For the formulation of Composition A, the corresponding amount of D-Glucopyranose, oligomeric, C10-16-alkyl glycosides, carboxymethyl ethers, sodium salts and D-Glucopyranose, oligomeric, C10-16-alkyl glycosides were filled into a vessel charged with the corresponding amount of deionized water. The mixture was stirred until complete homogenization of the surfactants and afterwards cocamidopropyl betaine was added into the mixture and stirred until complete homogenization. The formulation was preserved using a commercial preparation of sodium benzoate and potassium sorbate (Microcare SB) and the final pH was adjusted to pH=5.0-5.5 with citric acid.

### Preparation of Composition B (invention)

For the formulation of Composition B, the corresponding amount of D-Glucopyranose, oligomeric, C10-16-alkyl glycosides, carboxymethyl ethers, sodium salts and D-Glucopyranose, oligomeric, C10-16-alkyl glycosides were filled into a vessel charged with the corresponding amount of deionized water. The mixture was stirred until complete homogenization of the surfactants and afterwards cocamidopropyl betaine was added into the mixture and stirred until complete homogenization. Later, the corresponding amount of lauryl lactate was added and the formulation was stirred until complete homogenization. The formulation was preserved using a commercial preparation of sodium benzoate and potassium sorbate (Microcare SB) and the final pH was adjusted to pH=5.0-5.5 with citric acid.

Table 1 summarizes the results for the content of the compositions.

**Table 1:**

| Composition (% active wt) | Composition A (Comparative) | Composition B (Invention) |
|---|---|---|
| deionized water | Up to 100 | Up to 100 |
| D-Glucopyranose, oligomeric, C10-16-alkyl glycosides, carboxymethyl ethers, sodium salts | 3.1 | 3.1 |
| D-Glucopyranose, oligomeric, C10-16-alkyl glycosides | 3.3 | 3.3 |
| cocamidopropyl betaine | 4.0 | 4.0 |
| lauryl lactate | 0.0 | 0.8 |
| sodium chloride | 1.7 | 1.7 |
| citric acid | 1.3 | 1.2 |
| Preservative (Microcare SB) | 0.2 | 0.2 |

Viscosity of the cosmetic compositions prepared in Example 1 is summarized in Table 2 below. It can be observed that viscosity obtained in Composition B according to the invention is the desired viscosity for the cosmetic composition, showing higher viscosity values than comparison composition. Additionally, it can be seen that viscosity remains remarkably stable at different temperature conditions during 90 days. Furthermore, as observed from the foam evaluation, Composition B has a significant better perceived foam smoothness by panelist and foam volume does not decrease.

**Table 2:**

| Properties | Comparative | Invention |
|---|---|---|
| pH (100%) | 5.0 | 5.0 |
| Viscosity at 20°C, cP | 19 | 15600 |
| Viscosity at 20°C after 90 days at: | | |
| Temperature 5°C, cP | 18 | 14750 |
| Temperature 25°C, cP | 20 | 14520 |
| Temperature 40°C, cP | 20 | 14060 |
| Foam evaluation: | | |
| Foam smoothness | 3.0 | 3.5 |
| Foam volume | 3.0 | 3.0 |

Viscosity of the compositions was determined at 20°C, 24 hours after preparation, with a Brookfield viscometer model LV. Composition A viscosity was measured using a spindle number 2 at 60 rpm, for composition B viscosity was measured using a spindle number 4 at 12 rpm.

## Claims

1. A composition comprising:
a) at least an alkyl and/or alkenylglycoside derivative, and
b) at least a fatty acid ester derived from a hydroxycarboxylic and a fatty alcohol.

2. The composition according to claim 1, wherein the alkyl and/or alkenylglycoside derivative is represented by formula (I)
R¹-[G]ₚ-[A]ₙ Formula (I)
wherein
R¹ represents an alkyl or alkenyl group having from 4 to 24 carbon atoms, preferably from 10 to 18 carbon atoms, preferably from 10 to 16 carbon atoms, more preferably from 12 to 16 carbon atoms;
G is a sugar unit having 5 or 6 carbon atoms,
p is a number from 1 to 10,
n is a number from 1 to 4, and
A is selected from the group consisting of -(CH₂)ₘ-COOX, -C(=O)-CH₂-C(OH)(COOX)-CH₂-COOX, -C(=O)-CH(OH)-CH(OH)-COOX, -CH₂-CH(OH)-CH₂-S0₃X, -CH₂-CH(OH)-CH₂-C(=O)-CH₂-C(OH)(COOX)-CH₂-COOX, - P(O)(OX)₂, -CH₂-CH₂-SO₃X, and mixtures thereof, wherein m is a number from 1 to 5, and X is selected from the group consisting of hydrogen, an alkali metal, ammonium and alkaline earth metal.

3. The composition according to claim 1 to 2, wherein the alkyl and/or alkenylglycoside derivative is an alkyl and/or alkenylglycoside carboxylate represented by formula (II)
R¹-[G]ₚ-[(CH₂)ₘ-COOX]ₙ Formula (II)
wherein R¹, G, p, m, n and A are as defined in claim 2.

4. The composition according to claims 1 to 3, wherein the fatty acid ester derived from a hydroxycarboxylic acid and a fatty alcohol is represented by formula (III):
R³-COO-R⁴ Formula (III)
wherein R³ represents a hydroxyl-substituted alkyl or alkenyl group having 1 to 8 carbon atoms, optionally substituted by one or more -COOH groups, preferably wherein the hydroxyl group is attached to the carbon atom which is bonded to the -COO- moiety in formula (III), preferably selected from the group consisting of CH₂(OH)-, CH₃-CH₂(OH) - and COOH-CH₂-CH(CH₂-COOH)(OH)-; and
R⁴ represents an alkyl or alkenyl group having from 6 to 24 carbon atoms, preferably from 10 to 20 carbon atoms, more preferably from 10 to 16 carbon atoms, even more preferably from 12 to 16 carbon atoms

5. The composition according to claims 1 to 4, wherein the composition further comprises:
c) at least a alkyl and/or alkenylglycoside represented by formula (IV):
R¹-[G]ₚ Formula (IV)
wherein R¹, G and p are as defined in claim 2.

6. The composition according to claim 5, wherein the ratio by weight of components (a) : (c) is from 95:5 to 5:95, preferably from 80:20 to 20:80, more preferably from 60:40 to 40:60.

7. The composition according to claims 1 to 6, wherein the composition further comprises:
- d) at least an amphoteric surfactant.

8. The composition according to claim 7, wherein the ratio by weight of the sum of component (a) and component(c) to component (d) is from 90:10 to 10:90, preferably from 75:25 to 25:75, more preferably from 65:35 to 35:65.

9. The composition according to claims 5 to 8, wherein the ratio by weight of the sum of component (a) and component (c) to component (b) is from 98:2 to 31:69, preferably from 97:3 to 54:46, more preferably from 97:3 to 62:38.

10. The composition according to claims 1 to 9, wherein:
- component (a) is a compound of formula (II), wherein R¹ is a linear alkyl chain having from 10 to 16 carbon atoms, G represents a glucose, p is a number from 1 to 3, m is a number from 1 to 3, n is 1 to 4, and X represents an alkali metal;
- component (b) is selected from lauryl lactate and myristyl lactate;
- component (c), when present, is selected from the group consisting of caprylyl/caprylglycoside, myristylglycoside, laurylglycoside, cocoglycoside and mixtures thereof; and
- component (d), when present, is an alkyl amidopropyl betaine.

11. A process for producing the composition according to any one of claims 1 to 10, comprising the steps:
a. mixing the components (a) and (b) and, if present in the compositions, components (c) and/or (d), with water at a temperature suitable for homogeneization thereof, preferably at room temperature; and
b. stirring to obtain a homogeneous solution.

12. A process for producing the composition according to any one of claims 1 to 10, comprising the steps:
a. mixing components (a) and (b) and, if present in the compositions, components (c) and/or (d), without the presence of water to obtain a pre-mixture,
b. add the pre-mixture to water and stirring at a temperature suitable for homogeneization thereof.

13. A cosmetic composition comprising a composition according to claim 1 to 10 or a composition obtained by the process as defined in claims 11 to 12 and water.

14. The use of a cosmetic composition according to claim 13 for the care of skin and/or hair selected from the group consisting of cleansing of skin and/or hair, conditioning and moisturizing of skin and/or hair, and the dyeing of hair, preferably cleansing of skin and/or hair.

15. A method to cleanse skin and/or hair comprising the steps of wetting or dampening the skin and/or hair, applying the cosmetic composition according to claim 13 on the skin and/or hair, followed by rinsing.

16. A method to condition skin and/or hair comprising the steps of applying the cosmetic composition according to claim 13 on the wet skin and/or hair, followed by rinsing it off from the hair and/or skin with water, or alternatively be left on the hair and/or skin.
